# EUROPEAN PATENT APPLICATION

(11) **EP 3 804 645 A1**
(43) Date of publication of application: **14.04.2021**
(21) Application number: 19823325.6
(22) Date of filing: 08.01.2019
(51) Int. Cl.: A61B 17/86

(54) **ANTI-BURST HEADLESS COMPRESSION HOLLOW SCREW**

(30) Priority: 08.06.2018 CN 201810588522
(71) Applicant: Chuang Mei de Medical Device (Tianjin) Co., Ltd., Tianjin 300457 (CN)
(72) Inventor: LIU, Lina, Tianjin 300457 (CN); WANG, Tianxing, Tianjin 300457 (CN); PAN, Binghui, Tianjin 300457 (CN)
(74) Representative: Bandpay & Greuter
(86) International application number: PCT/CN2019/070778
(87) International publication number: WO 2019/242300

(57) **Abstract**

It's an explosion-proof headless compression cannulated screw comprising screw head, screw shaft, screw tail, and screw core hole. The screw core hole penetrates the screw head, screw shaft and screw tail. The outer surfaces of the screw head, screw shaft and screw tail are provided with threads, and the pitch of the above-mentioned threads decreases from the screw head to the screw tail. The screw head is provided with a self-tapping groove; the screw tail is provided with an inner hexagonal hole or an inner hexagonal flower-shaped hole; the thread on the outer surface of the screw tail is provided with multiple pressure relief and explosion-proof grooves. The present invention continuously pressurizes during the screwing in process, thereby obtaining a better pressurizing effect. The screw head is provided with a self-tapping groove, which replaces the screw tip with a self-tapping groove, which reduces the irritation of soft tissues and can realize the self-tapping and self-drilling. The pressure relief and explosion-proof groove at the screw tail can make screwing in easier on the one hand, and avoid bone burst during screwing on the other hand, which simplifies the operation and facilitates the recovery of patients after surgery.

## Description

### Technical Field

The invention relates to the technical field of orthopedic medical devices, in particular to an explosion-proof headless compression cannulated screw.

### Background technique

Fracture is one of the most common injuries. As the population ages, the annual incidence of fractures is gradually increasing. Surgery is a relatively effective treatment protocol. With the continuous development of new surgical theories, people realize that the degree of damage to the soft tissue around the fracture and it is equally important to ensure the bone tissue has a good blood supply. The headless compression cannulated screw turn out to be a good solution to reduce the stimulation of the soft tissue.

The headless compression cannulated screw in the existing techniques includes a screw head, a screw shaft, a screw tail, and a screw core hole. A uniform continuous thread is provided on the outside of the screw, and the screw head is provided with a screw tip. During use, special drilling tools are used to drill holes in bone tissue, and then it is the screw tip that guides it screwed in. However, the existing headless compression cannulated screws cannot achieve effective compression, and it is easy to stimulate the soft tissue around the bone, which is difficult to screw in. There is a risk of bone bursting caused by the enlarged diameter of screw tail.

### Invention contents

The technical problem to be solved by the present invention is to provide an explosion-proof headless compression cannulated screw. The technical solution adopted by the present invention to solve the technical problems existing in the known technology is:
The explosion-proof headless compression cannulated screw, the present invention, comprises screw head, screw shaft, screw tail, and screw core hole. The screw core hole penetrates the screw head, screw shaft and screw tail. The outer surfaces of the screw head, screw shaft and screw tail are provided with threads, and the pitch of the above-mentioned threads decreases from the screw head to the screw tail. The screw head is provided with a self-tapping groove; the screw tail is provided with an inner hexagonal hole or an inner hexagonal flower-shaped hole, and the thread on the outer surface of the screw tail is provided with multiple pressure relief and explosion-proof grooves.

The present invention can also be realized with the following measures:
The pitch of the thread is gradually and continuously reduced from the screw head and the screw shaft to the screw tail.

Six pressure relief and explosion-proof grooves are evenly arranged on the threads on the outer surface of the screw tail.

The advantages and positive effects of the present invention are:
In the explosion-proof headless compression cannulated screw of the present invention, the thread pitch from the head to the tail of the screw is gradually reduced, and the screw is continuously pressurized during the screwing process, thereby obtaining a better compression effect. The screw head is provided with self-tapping grooves which replace the screw tip, which reduces the stimulation to soft tissues. This design can realize the self-tapping and self-drilling of the screw. No other drilling tools are required during use. The pressure relief and explosion-proof groove at the end of the screw can make it easier to screw in. On the other hand, it avoids the bone burst during the screwing process, which simplifies the operation and facilitates the recovery of the patient after surgery.

### Figures

Figure 1 is a front view of the explosion-proof headless compression cannulated screw of present invention.
Figure 2 is the left view of the explosion-proof headless compression cannulated screw of present invention.
Figure 3 is the right view of the explosion-proof headless compression cannulated screw of present invention.

### Detailed description of the present invention

The following contents are detailed description of the present invention.

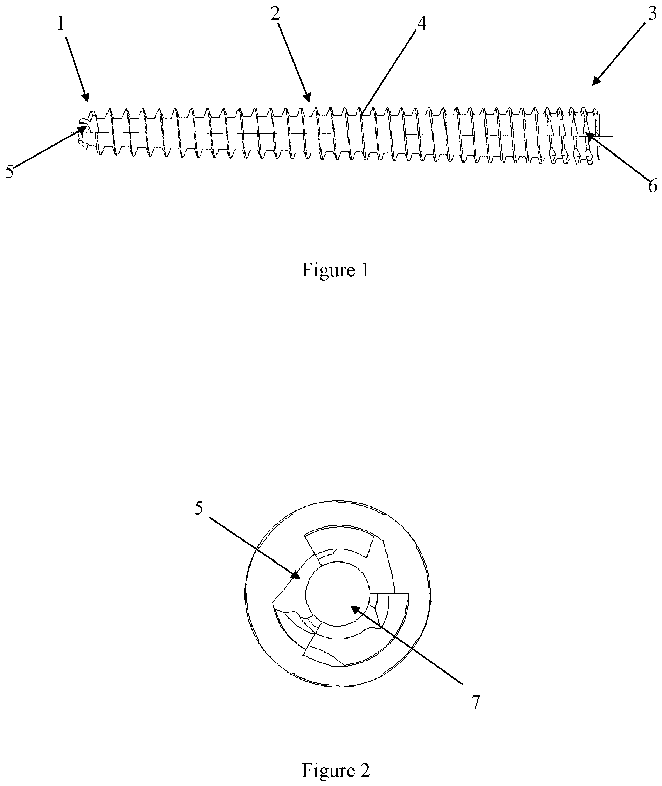

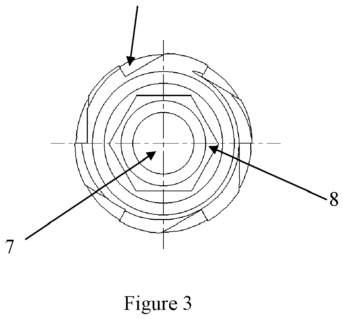

As shown in Figure 1 to figure 3, the explosion-proof headless compression cannulated screw of present invention comprises screw head 1, screw shaft 2, screw tails 3, and screw core hole 7. The screw core hole penetrates screw head, screw shat and screw tail, which makes the guide wire locate more accurately when going through the screw core hole during surgery. The outer surfaces of the screw head, screw shaft and screw tail are provided with threads 4. The pitch of the above-mentioned threads decreases from the screw head to the screw tail. In other words, the screw head has the largest pitch. When the screw is used, the larger pitch of the screw head is more conducive to the screw insertion, while the smaller pitch of the screw tail is more conducive to the compression and fixation of the screw and bone tissue. The screw head is provided with a self-tapping groove 5, which can help realize the self-tapping and self-drilling of the screw. For those small models, other drilling tools are no longer needed; for those large models, drill holes first, and then implant. Even though, it would be much easier by using tools to screw in for screws with self-tapping grooves than those which don't. The screw can be completely embedded in the bone without adversely affecting the movement of the joint and reduces the stimulation of the soft tissue surrounding the bone. The screw tail is provided with an inner hexagonal hole 8 or an inner hexagonal flower-shaped hole. Both of them have the same function. They can be implanted into the bone by using tools. The specific shape can be selected according to the actual situation. The thread on the outer surface of the screw tail is provided with multiple pressure relief and explosion-proof grooves 6, so that the pressure strength between the radial direction of the screw and the bone tissue can be reduced without reducing the axial compression strength of the screw as much as possible. The screw is easier to screw in, and it also avoids the damage caused by bone burst.

The pitch of the thread is gradually and continuously reduced from the screw head and the screw shaft to the screw tail, which makes the processes of screwing in and pressurizing more evenly and smoothly.

Six pressure relief and explosion-proof grooves are evenly arranged on the threads on the outer surface of the screw tail. The setting position of the grooves can correspond to the setting of the hexagonal hole or the hexagonal flower-shaped hole at the screw tail.

The above are only the preferred embodiments of the present invention, and do not limit the present invention in any form. Although the present invention has been disclosed as above in preferred embodiments, it is not intended to limit the present invention. Any person familiar with the profession, without departing from the scope of the technical solution of the present invention, will of course use the disclosed technical content to make some changes or modifications to become equivalent embodiments with equivalent changes. However, without departing from the content of the technical solution of the present invention, any simple modifications, equivalent changes and modifications made to the above embodiments based on the technical essence of the present invention shall fall within the scope of the technical solution of the present invention.

## Claims

1. It's an explosion-proof headless compression cannulated screw comprising screw head, screw shaft, screw tail, and screw core hole. The screw core hole penetrates the screw head, screw shaft and screw tail. Here are the features:
The outer surfaces of the screw head, screw shaft and screw tail are provided with threads, and the pitch of the above-mentioned threads decreases from the screw head to the screw tail. The screw head is provided with a self-tapping groove; the screw tail is provided with an inner hexagonal hole or an inner hexagonal flower-shaped hole. The thread on the outer surface of the screw tail is provided with multiple pressure relief and explosion-proof grooves.

2. According to claims 1, the features of above explosion-proof headless compression cannulated screw lay in: The pitch of the thread gradually decreases from the screw head, the screw shaft to the screw tail.

3. According to claim 1 or 2, the features of above explosion-proof headless compression cannulated screw lay in: Six pressure relief and explosion-proof grooves are evenly arranged on the threads on the outer surface of the screw tail.
